(19) Europäisches Patentamt
European Patent Office
Office européen des brevets

(11) Veröffentlichungsnummer: **0 039 880**
**B2**

(12) # NEUE EUROPÄISCHE PATENTSCHRIFT

(45) Veröffentlichungstag der neuen Patentschrift: **03.06.87**

(51) Int. Cl.⁴: **C 12 Q 1/34**

(21) Anmeldenummer: **81103385.1**

(22) Anmeldetag: **05.05.81**

(54) **Mittel zum Nachweis esterolytischer und/oder proteolytischer Enzyme und dafür als Substrate geeignete Phenoxy-Aminosäure- und Phenoxy-Peptidester, Verfahren zu deren Herstellung sowie deren Verwendung zur Herstellung der erfindungsgemässen Mittel.**

(30) Priorität: **09.05.80 DE 3017721**

(43) Veröffentlichungstag der Anmeldung: **18.11.81 Patentblatt 81/46**

(45) Bekanntmachung des Hinweises auf die Patenterteilung: **29.06.83 Patentblatt 83/26**

(45) Bekanntmachung des Hinweises auf die Entscheidung über den Einspruch: **03.06.87 Patentblatt 87/23**

(84) Benannte Vertragsstaaten: **AT BE CH DE FR GB IT LI LU NL SE**

(56) Entgegenhaltungen:
**EP - A - 0 012 957**
**EP - A - 0 014 929**
**DE - A - 2 531 539**
**DE - C - 2 130 559**
**DE - C - 2 240 471**
**DE - C - 2 633 087**
**GB - A - 1 128 371**
**US - A - 3 905 872**

**W.J. WILLIAMS et al.: "Haematology", 2. Ausgabe, 1977, Kapitel A25 "Leukocyte esterases", Seiten 1633-1636 New York, U.S.A:**
**(Gössner) Histochemie Bd. 1, S. 48-96 (1958)**
**(Sweetman) J. Histochem./Cytochem. Bd. 22, Nr. 5, S. 327-339 (1974)**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Berger, Dieter, Dr. rer. nat., D-6806 Bensheimer-Strasse 45, Viernheim (DE)**
Erfinder: **Frey, Günter, D-6701 Pfalzgrafenstrasse 7, Ellerstadt (DE)**
Erfinder: **Knappe, Wolfgang-Reinhold, Dr. rer. nat., D-6842 Karlsbader-Strasse 3, Bürstadt (DE)**
Erfinder: **Kuhr, Manfred, Dr. rer. nat., D-6800 Werthmannweg 23, Mannheim 31 (DE)**
Erfinder: **Rittersdorf, Walter, Dr. rer. nat., D-6800 Kasseler Strasse 6, Mannheim 31 (DE)**
Erfinder: **Werner, Wolfgang, Dr. rer. nat., D-6800 Meissener Weg 39, Mannheim 31 (DE)**

(56) Entgegenhaltungen: (Fortsetzung)
**(Baggiolini); Annals NY Acad. of Sciences, Bd. 256, S. 261-265 (1975)**
**(Ornstein) Abstracts Histochem. Soc. S. 411 (1973)**
**(Starkey) Biochem. J. Bd. 155, S. 265 (1976)**
**(Pearse) Int.Rev.Cytol. Bd. 3, S. 329 (1954)**
**(Li) J. Cytochem/Histochem. Bd. 21 (1) S. 1-12 (1973)**
**Woroshzow: Grundlagen ... (1966) S. 511**
**(Bayer) Chem. Berichte Bd. 16, S. 2188-2204 (1883)**
**(Martinet) Comptes Rendus 8. März 1920 S. 593-597**
**(Holt) J. Cell-Biology Bd. 29, S. 361-366 (1966)**
**(Gossrau) Histochem. Bd. 57, S. 323-342 (1978)**
**(Schmalzel) Klin. Wochenschrift Bd. 46(12), S. 642-650 (1978)**
**(Sweetman) J. Histochem./Cytochem. Bd. 22, S. 327-339 (1974)**
**(Janoff) Biolog. J. Bd. 114(1), S. 157-159 (1969)**



ACTORUM AG

**Beschreibung**

In der Diagnostik der Erkrankungen der Niere und des Urogenitaltraktes kommt dem Nachweis von Leukozyten im Urin eine grosse Bedeutung zu. Bisher wurde dieser Nachweis mikroskopisch durchgeführt, indem die Zahl der Leukozyten, die sich in einem bestimmten Urinvolumen befanden, unter dem Mikroskop ausgezählt wurde. Diese Methode ist jedoch sehr zeitaufwendig, mühevoll und ermüdend und erfordert darüberhinaus den Einsatz von geschultem Personal.

Seit einiger Zeit wird daher versucht, als Nachweisprinzip für Leukozyten in verschiedenen Körperflüssigkeiten enzymatische Reaktionen heranzuziehen, da die Leukozyten ein breitgefächertes Enzymspektrum besitzen.

Mittel zum Nachweis von Leukozyten in Körperflüssigkeiten, bei denen die in den Leukozyten vorhandene esterolytische und/oder proteolytische Aktivität für analytische Zwecke ausgenutzt werden, sind aus den deutschen Offenlegungsschriften 2 826 965 und 2 836 644 bekannt. Sulfonphthaleinester bzw. Azo-Farbstoffester werden dabei als Substrate für die Leukozyten-Esterasen und/oder -Proteasen verwendet. Die bei der enzymatischen Umsetzung freigesetzten Farbstoffe werden nach allgemein bekannten Methoden ausgewertet. Die in diesen Schriften beschriebenen Mittel sind noch zu unempfindlich. Sie weisen zu lange Reaktionszeiten für die untere Nachweisgrenze auf, so dass die praktische Anwendung noch mit gewissen Nachteilen, vor allem zu langen Wartezeiten bei der Durchführung des Testes, verbunden ist.

Verschiedene Methoden für den Nachweis von Proteasen und Esterasen sind auch aus der Histo- und Cytochemischen Enzymologie bekannt (vgl. beispielsweise A.G.E. Pearse, Histochemistry, Theoretical and Applied, 3. Ed., Churchill Livingstone, Edinburgh-London-New York 1968). Im Prinzip werden dabei ebenfalls farblose oder schwach gefärbte Ester eingesetzt, die durch die enzymatische Spaltung zumeist in eine farblose Säure und eine ebenfalls farblose Alkohol-(Phenol)-Komponente zerfallen. Letztere wird dann in einer der enzymatischen Verseifung folgenden Reaktion zu farbigen Produkten umgesetzt, z.B. durch Kupplung mit Diazoniumsalzen oder oxidative Reaktion.

F. Schmalzl und H. Braunsteiner beschreiben zum Beispiel in Klin. Wschr. 46, 642 (1968) einen spezifischen Cytochemischen Leukozytenesterasenachweis mit Naphthol-AS-D-Chloracetat als Substrat und einem Diazoniumsalz zur Bildung der farbigen Azo-Verbindung.

Für ein Mittel zum schnellen und einfachen Nachweis von Leukozyten in Körperflüssigkeiten, wie z.B. im Harn, erwiesen sich Zweikomponenten-Systeme dieser Art als nicht geeignet. Sie reagieren viel zu unempfindlich. Beispielsweise zeigen Proben mit 5000 Leukozyten/µl keine Reaktion. Darüberhinaus reagieren bekanntlich viele im Harn vorkommende Verbindungen, wie Urobilinogen, Stercobilinogen, Bilirubin und andere, mit Diazoniumsalzen. Dies beweisen am besten die im Handel befindlichen patentierten Teste für den Nachweis von Urobilinogen oder Bilirubin im Harn, die für die Nachweisreaktion Diazoniumsalze verwenden. Die in der Literatur für den Esterase-Nachweis eingesetzten Diazoniumsalze zeigen die beschriebene Nebenreaktion mit anderen Harnbestandteilen und sind z.T. auch wegen ihrer Eigenfarbe für einen Leukozytentest nicht brauchbar.

F. Sweetman und L. Ornstein beschreiben in J. Histochem./Cytochem. 22, 327–339 (1974) ebenso wie Ornstein et al. in Abstracts Histochem. Soc. 1973, S. 411 die Anfärbung verschiedener esterolytischer und proteolytischer Enzyme auf Elektrophorese-Platten, die zur Reinigung und Separierung der Enzyme dienen. Angewandt wird dabei die Kupplung verschiedener Naphthylester, u.a. 1-Naphthyl-N-acetyl-D,L-alanin mit diazotiertem Pararosanilin. Hierbei werden trotz der hohen Ausgangskonzentration der Enzyme, die aus 40–100 Millionen Zellen/ml gewonnen werden, und der Abtrennung von störenden Nebenbestandteilen Reaktionszeiten von über zwei Stunden für eine Anfärbung benötigt.

P.M. Starkey und A.J. Barrett beschäftigen sich in Biochem. J. 155, 265–271 (1976) mit der Charakterisierung einer aus menschlichen Milzzellen isolierten und rein dargestellten Elastase. Die Spaltungsaktivität dieses Enzyms gegenüber verschiedenen Naphthylestern und Nitroanilinderivaten wird beschrieben. Aus immunologischen Untersuchungen wird der Schluss gezogen, dass diese Elastase mit derjenigen aus menschlichen neutrophilen Leukozyten übereinstimmt. Die Autoren führen ihre Untersuchungen lediglich im Hinblick auf die chemischen Eigenschaften von Leukozyten-Enzymen, nicht aber zum Nachweis unbekannter Mengen solcher Enzyme durch.

Es sind ferner verschiedene Esterase-Nachweisprinzipien auf Basis eines Puffers, eines bestimmten Diazoniumsalzes sowie eines geeigneten Esters bekannt. Beispielsweise wird in W.J. Williams et al., Haematology, 2. Ausgabe 1977, Karpitel A25: Leukocyte esterases, Seiten 1633–1636, Naphtholchloracetat in Verbindung mit mehrfach diazotierten Farbstoffen und einem Phosphatpuffer zum Nachweis einer Esterase-Aktivität beschrieben. In dem US-Patent 3 905 872 wird eine Kombination von Naphthylphosphat oder Phenylphosphat mit einem Diazoniumsalz zur Bestimmung der alkalischen Phosphatase beansprucht. All diese vorbekannten Esterase-Nachweissysteme haben sich jedoch zum Nachweis der esterolytischen und/oder proteolytischen Enzymaktivität der Leukozyten in Körperflüssigkeiten nicht als brauchbar erwiesen.

Aufgabe der vorliegenden Erfindung war es nun, ein Mittel zum Nachweis esterolytischer und/oder proteolytischer Enzyme mit einer Kombination von Estern und Diazoniumsalzen als Reaktionspartner für diese Enzyme bereitzustellen, mit dem diese auf einfache und leicht zu

handhabende Weise in kürzerer Zeit nachgewiesen werden können und die keine störenden Nebenreaktionen mit anderen Bestandteilen der untersuchten Probe aufweisen.

Diese Aufgabe wurde dadurch gelöst, dass eine bestimmte Kombination von geeigneten Estern und speziell substituierten Diazoniumsalzen eingesetzt wird, wobei überraschenderweise die verwendeten Diazoniumsalze keine Nebenreaktionen mit anderen Harnbestandteilen wie beispielsweise Bilirubin und Urobilinogen eingehen, sondern spezifisch und schnell die bei der Spaltung der eingesetzten Ester entstehenden phenolischen Komponenten unter Bildung einer farbigen Verbindung kuppeln.

Die geeigneten Ester müssen gegenüber den esterolytischen und/oder proteolytischen Enzymen hinreichend reaktiv sein, damit sie möglichst rasch in die Säure- und Alkohol-Komponente (worunter auch Phenole verstanden werden) gespalten werden. Die Ester sind auch so auszuwählen, dass die freigesetzten Alkohol-Komponenten gut und vollständig mit den eingesetzten Diazoniumsalzen kuppeln. Die Reaktivität der Diazoniumsalze muss durch geeignete Auswahl der Substituenten derart abgestimmt sein, dass zwar eine hinreichend rasche Kupplung mit den aus den erfindungsgemäss verwendeten Estern freigesetzten Alkohol-Komponenten, jedoch keine Reaktion mit sonstigen Bestandteilen der Testlösung stattfindet.

Gegenstand der vorliegenden Erfindung ist die Verwendung von Mitteln zum Nachweis esterolytischer und/oder proteolytischer Enzyme, bestehend aus einem saugfähigen Träger, einer Filmschicht, einer Pulvermischung, einem Lyophilisat, einer Lösung oder einer Reagenztablette, enthaltend ein geeignetes Esterase- und/oder Protease-Nachweisprinzip, einen Puffer sowie gegebenenfalls weitere, üblicherweise verwendete Zusatzstoffe zum Nachweis von Leukozyten im Urin, wobei das Mittel dem zu untersuchenden Urin zugegeben wird und die entstehende Farbbildung visuell oder photometrisch beurteilt wird, dadurch gekennzeichnet, dass als Esterase- und/oder Protease-Nachweisprinzip eine Kombination aus einem speziell substituierten Diazoniumsalz und einem geeigneten Ester eingesetzt wird, deren Reaktivität durch geeignete Auswahl der Substituenten derart aufeinander abgestimmt wird, dass zwar eine hinreichend rasche Kupplung mit der aus dem Ester freigesetzten Alkohol-Komponente, jedoch keine Reaktion mit sonstigen Bestandteilen der Testlösung erfolgt, wobei der Ester aus der Gruppe der Verbindungen der allgemeinen Formel II

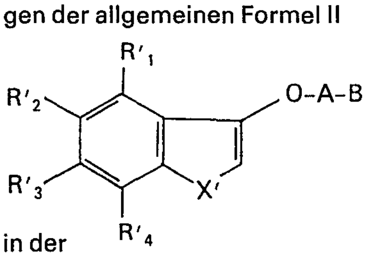

(II)

in der

$R'_1$, $R'_2$, $R'_3$, $R'_4$, die jeweils gleich oder verschieden sein können, jeweils ein Wasserstoff- oder ein Halogenatom, eine niedere Alkyl-, eine niedere Alkoxy-, eine Aryl-, eine Aralkyl-, eine Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylamino-Gruppe oder jeweils zwei benachbarte Substituenten einen gegebenenfalls durch Halogen substituierten benzoannellierten Rest,

X′ ein Schwefelatom oder eine gegebenenfalls durch einen niederen Alkyl-, einen Aryl-, einen Aralkyl- oder einen Acylrest substituierte Iminogruppe,

A einen Aminosäure- oder einen Peptidrest,

B eine in der Peptidchemie übliche oder davon abgeleitete Stickstoffschutzgruppe

bedeuten, oder der Verbindungen der allgemeinen Formel III

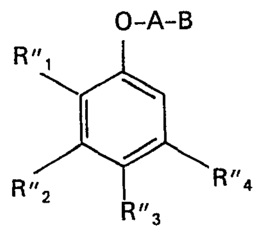

(III),

in der

$R_1''$, $R_2''$, $R_3''$, $R_4''$, die gleich oder verschieden sein können, jeweils Wasserstoff, eine niedere Alkyl-, eine niedere Alkoxy-, Alkylamino- oder Dialkylaminogruppe bedeuten, wobei zwei benachbarte Substituenten auch einen annellierten Benzolring vorstellen können,

A und B die oben angegebene Bedeutung haben,

ausgewählt wird.

Ein weiterer Gegenstand der vorliegenden Erfindung sind Mittel zum Nachweis esterolytischer und/oder proteolytischer Enzyme in Leukozyten im Urin, bestehend aus einem saugfähigen Träger, einer Filmschicht, einer Pulvermischung, einem Lyophilisat, einer Lösung oder einer Reagenztablette, enthaltend ein geeignetes Esterase- und/oder Protease-Nachweisprinzip, einen Puffer sowie gegebenenfalls weitere, üblicherweise verwendete Zusatzstoffe, dadurch gekennzeichnet, dass als Esterase- bzw. Protease-Nachweisprinzip eine Kombination aus einem speziell substituierten Diazoniumsalz und einem geeigneten Ester eingesetzt wird, deren Reaktivität durch geeignete Auswahl der Substituenten derart aufeinander abgestimmt wird, dass zwar eine hinreichend rasche Kupplung mit der aus dem Ester freigesetzten Alkohol-Komponenten, jedoch keine Reaktion mit sonstigen Bestandteilen der Testlösung erfolgt, wobei der Ester aus der Gruppe der Verbindungen der allgemeinen Formel II

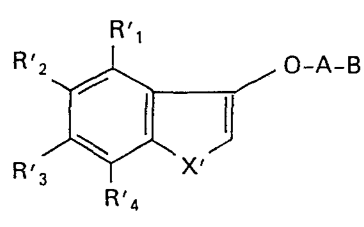

(II),

in der

$R'_1$, $R'_2$, $R'_3$, $R'_4$, die jeweils gleich oder verschieden sein können, jeweils ein Wasserstoff- oder ein Halogenatom, eine niedere Alkyl-, eine niedere Alkoxy-, eine Aryl-, eine Aralkyl-, eine Aralkoxy-, eine Hydroxy-, eine Carboxy-, eine Carboxy-nieder-Alkoxy, eine Aralkoxycarbonyl-, eine Aralkoxycarbonyl-nieder-Alkoxy- eine Nitro- oder eine niedere Acylamino-Gruppe oder jeweils zwei benachbarte Substituenten einen gegebenenfalls durch Halogen substituierten benzoannellierten Rest,

X' ein Schwefelatom oder eine gegebenenfalls durch einen niederen Alkyl-, einen Aryl-, einen Aralkyl- oder einen Acylrest substituierte Iminogruppe,

A einen Aminosäure- oder einen Peptidrest,

B eine in der Peptidchemie übliche oder davon abgeleitete Stickstoffschutzgruppe bedeuten,

oder der Verbindungen der allgemeinen Formel III

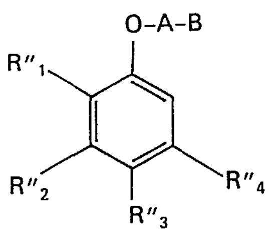

(III),

in der

$R''_1$, $R''_2$, $R''_3$, $R''_4$, die gleich oder verschieden sein können, jeweils Wasserstoff, eine niedere Alkyl-, eine niedere Alkoxy-, Alkylamino- oder Dialkylamino-Gruppe bedeuten, wobei zwei benachbarte Substituenten auch einen annellierten Benzolring vorstellen können,

A und B die oben angegebene Bedeutung haben, wobei wenn $R_1''$ und $R_2''$ oder $R_2''$ und $R_3''$ ein annellierter Benzolring ist, B nicht eine Acetyl- oder Benzyloxycarbonylgruppe darstellt, ausgewählt wird.

Geeignet substituierte Diazoniumsalze im Sinne der vorliegenden Erfindung sind beispielsweise Verbindungen der allgemeinen Formel I

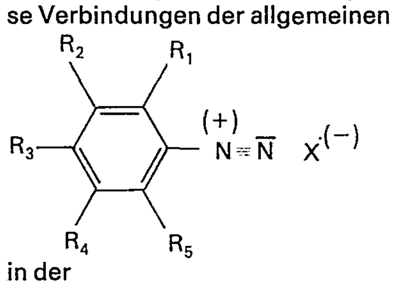

(I),

in der

$R_1$ eine niedere Alkyl-, eine niedere Alkoxy-, eine niedere Alkylmercapto-, eine N-Morpholino-, eine N-Thio-morpholino-, eine N-Pyrrolidino-, eine gegebenenfalls N'-alkylierte N-Piperazino-, eine N-Piperidino-Gruppe, Halogen oder Wasserstoff,

$R_3$ eine niedere Alkyl-, eine niedere Alkoxy-, eine Aryloxy-, eine niedere Alkylmercapto-, Alkylamino-, Dialkylamino-, eine Hydroxy-, eine N-Morpholino-, N-Thio-morpholino-, N-Pyrrolidino-, eine gegebenenfalls N'-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasserstoff,

$R_2$, $R_4$, $R_5$, die gleich oder verschieden sein können, jeweils eine niedere Alkyl-, eine niedere Alkoxy-, eine niedere Alkylmercapto-Gruppe, Halogen oder Wasserstoff, wobei $R_1$ und $R_2$ zusammen einen annellierten Benzolring vorstellen können, und

X ein stabilisierendes Anion bedeuten.

Die Ester der allgemeinen Formel II und III werden schnell und vollständig von Esterasen und/oder Proteasen, beispielsweise von den in den neutrophilen Granulozyten vorkommenden Esterasen und Proteasen verseift. Die freigesetzten Phenolkomponenten reagieren gut, schnell und selektiv mit den wenig elektrophilen Diazoniumsalzen der allgemeinen Formel I. Es lassen sich daher nach dem erfindungsgemässen Prinzip stabile und schnell anzeigende Mittel für den Nachweis von Leukozyten in Körperflüssigkeiten herstellen. Darüberhinaus hat es sich gezeigt, dass sich die erfindungsgemässen Mittel auch ausgezeichnet zum allgemeinen Nachweis proteolytischer Enzyme, wie beispielsweise von Elastase, Chymotrypsin oder Trypsin in wässrigen Lösungen bzw. in Körperflüssigkeiten, wie z.B. Vollblut, Serum und Liquor, Pankreassekret oder wässrigen Stuhlextrakten eignen.

Die Diazoniumsalze der allgemeinen Formel I sind teilweise bekannte Verbindungen. Derivate, in denen der Substituent $R_1$ und/oder $R_3$ eine N-Thio-morpholino-, N-Pyrrolidino-, eine gegebenenfalls N'-alkylierte N-Piperazino- oder N-Piperidino-Gruppe vorstellen, sind neue Verbindungen. Sie können jedoch in Analogie zu den bekannten Substanzen nach an sich bekannten Methoden hergestellt werden.

Die Verbindungen der allgemeinen Formel II sind in der deutschen Offenlegungsschrift 2 854 987 beschrieben.

Die Ester der allgemeinen Formel III sind dann neue Verbindungen, wenn $R_1''$ und $R_2''$ oder $R_2''$ und $R_3''$ ein annellierter Benzolring ist, B nicht eine Acetyl- oder Benzyloxycarbonylgruppe darstellt. Sie können hergestellt werden, indem man Verbindungen der allgemeinen Formel IV

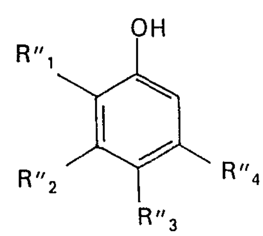

(IV),

in der

$R''_1$, $R''_2$, $R''_3$ und $R''_4$ die oben angegebene Bedeutung haben,

mit Aminosäuren und Peptiden der allgemeinen Formel V

HO–A–B (V),

in der

A und B die oben angegebene Bedeutung haben,

bzw. geeigneten reaktiven Derivaten hiervon nach in der Peptidchemie üblichen Methoden umsetzt.

Als reaktive Derivate werden z.B. die Säurechloride, beziehungsweise die bei der Peptidsynthese üblicherweise verwendeten Mischanhydride, beispielsweise mit Chlorameisensäureethylester, oder Aktivester eingesetzt.

Ein weiterer Gegenstand der vorliegenden Anmeldung sind daher die neuen Ester der allgemeinen Formel III sowie deren Verwendung zur Herstellung von Mitteln zum Nachweis esterolytischer und/oder proteolytischer Enzyme.

Unter Halogen in der Definition von $R_1$, $R_2$, $R_3$, $R_4$ und $R_5$ bzw. $R'_1$, $R'_2$, $R'_3$ und $R'_4$ ist Fluor, Chlor, Brom und Jod, vorzugsweise Chlor und Brom zu verstehen.

Die niedere Alkyl-, Alkoxy-, Alkylmercapto-, Alkyl- und Dialkylamino-Gruppen in den Definitionen von $R_1$– $R_5$, $R'_1$– $R'_4$ sowie $R''_1$– $R''_4$ enthalten 1 bis 5, vorzugsweise 1 bis 3 Kohlenstoffatomen, wobei die entsprechenden Methylreste ganz besonders bevorzugt sind.

Als stabilisierendes Anion X ist Tetrafluoroborat, Tetrachlorozinkat bzw. Perchlorat bevorzugt.

Unter einer Aralkoxygruppe in der Definition von $R'_1$, $R'_2$, $R'_3$ und $R'_4$ sowie einer Aralkylgruppe in der Definition von X′ sind z.B. durch Oxynieder-Alkyl bzw. niedere Alkylreste substituierte Phenyl- und Naphthylgruppen zu verstehen, wobei der Alkylrest 1 bis 5, vorzugsweise 1 bis 3 Kohlenstoffatome enthält. Besonders bevorzugt sind der Benzyloxy- bzw. der Benzylrest.

Als niedere Acylaminogruppe von $R'_1$, $R'_2$, $R'_3$ und $R'_4$ kommen die Amidgruppierungen der niederen aliphatischen Carbonsäuren mit 1 bis 5, vorzugsweise 1 bis 3 Kohlenstoffatomen, in Frage. Besonders bevorzugt ist der Acetylaminorest.

Als Acylrest in der Definition von X′ kommen die Reste aliphatischer Carbonsäuren mit 1 bis 5, vorzugsweise 1 bis 3 Kohlenstoffatomen, oder auch aromatischer Carbonsäuren, wie beispielsweise der Benzoe- oder Naphthoesäuren, in Frage. Besonders bevorzugt sind Acetyl- und Benzoylreste.

Unter einem Aryl- bzw. Aralkylrest in der Definition von $R'_1$, $R'_2$, $R'_3$, $R'_4$ und X′ sind vorzugsweise die Phenyl- oder Naphthylgruppe bzw. der Benzylrest zu verstehen.

Als Aminosäurerest in der Definition von A kommen vorzugsweise die Reste der natürlichen α-Aminosäuren in ihrer L- oder D-Form oder auch in ihrer racemischen Form, in Frage. Besonders bevorzugt sind die Reste von Glycin, Alanin, Valin, Leucin, Isoleucin, Phenylalanin und Tyrosin, wobei jeweils die L-Form ganz besonders bevorzugt ist. Gegebenenfalls vorhandene freie Hydroxygruppen können acyliert, vorzugsweise acetyliert, sein.

Unter einem Peptidrest in der Definition von A sind z.B. Di-, Tri-, Tetra- und Pentapeptide, vorzugsweise Di- und Tripeptide, zu verstehen, wobei als Aminosäure-Komponenten vorzugsweise die oben erwähnten Aminosäuren Verwendung finden.

Als in der Peptidchemie übliche Stickstoffschutzgruppe in der Definition von B sind z.B. Acyl-, Oxycarbonyl-, Thiocarbonyl-, Sulfonyl-, Sulfenyl-, Vinyl-, Cyclohexenyl-, Phosphoryl- oder Carbamoyl-Gruppen zu verstehen.

Die erfindungsgemäss verwendeten Diazoniumsalze der allgemeinen Formel I und die Ester der allgemeinen Formel II und III werden in Konzentrationen von $10^{-4}$ mol/l bis $10^{-1}$ mol/l vorzugsweise $10^{-3}$ mol/l bis $10^{-2}$ mol/l Imprägnierlösungs-Beschichtungsmasse oder zu untersuchende Flüssigkeit eingesetzt.

Ein weiterer Bestandteil des erfindungsgemässen Mittels zum Nachweis proteolytischer Enzyme und insbesondere der Leukozyten-Proteasen ist ein geeignetes Puffersystem. Hierzu kommen z.B. Phosphat-, Borat-, Barbiturat-, Tris-(hydroxymethyl)-aminomethan-(=Tris), 2-Amino-2-methyl-orpandiol-1,3-(=Amediol)- oder Aminosäure-Puffer in Frage, wobei pH-Wert und Kapazität so gewählt werden müssen, dass sich in der Messlösung bzw. auf dem Teststreifen ein pH-Wert von 6–10, vorzugsweise von 7–9, einstellt.

Ein weiterer Bestandteil des erfindungsgemässen Mittels für den Nachweis proteolytischer Enzyme kann ein Netzmittel sein, da hierdurch eine homogenere Farbverteilung und z.T. brillantere Farben erzielt werden können. Es können kationen-, aber auch anionenaktive, sowie amphotere und nichtionogene Netzmittel in Konzentrationen von 0,05–2% (w/v), vorzugsweise 0,1–1% (w/v) eingesetzt werden.

Als weiterer Bestandteil des erfindungsgemässen Mittels kann als Stabilisator ein Phosphor- bzw. Phosphonsäureamid der allgemeinen Formel VI dienen

$$\begin{array}{c} R_6 \\ | \\ R_7 - P = O \\ | \\ R_8 \end{array} \qquad (VI),$$

in der

$R_6$ eine Dialkylamino-, eine Alkoxy-, eine Aryloxy-, eine Alkyl- oder eine Arylgruppe oder einen N-Morpholinrest und

$R_7$ und $R_8$ eine Dialkylaminogruppe oder einen N-Morpholinrest bedeuten.

Als Alkoxy- bzw. Alkylgruppe in der Definition von $R_6$ kommen Kohlenwasserstoffreste bis 10 Kohlenstoffatomen in Frage.

Unter Aryl- bzw. Aryloxygruppe in der Definition von $R_6$ sind gegebenenfalls durch Halogen, niedere Alkyl- oder Alkoxygruppen substituierte Phenyl- oder Napthylreste zu verstehen.

Mit Hilfe der Verbindungen der allgemeinen Formel VI gelingt eine erstaunliche Stabilisierung der Rezepturen.

Die Phosphor- und Phosphonsäureamide der allgemeinen Formel VI sind bekannte Verbindungen, sie finden z.B. in der deutschen Auslegeschrift 2 235 127 Anwendung als Stabilisatoren von Teststreifenrezepturen, die auf der Basis eines Peroxidasenachweises arbeiten.

Die Stabilisatoren des Phosphor- und Phosphonsäureamid-Typs der allgemeinen Formel VI werden der wässrigen oder bevorzugt der organischen Imprägnierlösung in Konzentrationen von 1–20% (w/v), vorzugsweise 5–15% (w/v), zugesetzt.

Überraschenderweise wurde gefunden, dass die Reaktionszeiten des erfindungsgemässen diagnostischen Mittels zum Nachweis proteolytischer Enzyme, insbesondere proteolytischer Leukozytenenzyme, erheblich verkürzt werden können, wenn zusätzlich zu den Diazoniumsalzen, Estern und bisher angeführten Hilfsstoffen ein oder mehrere Aktivatoren eingesetzt werden. Als für das erfindungsgemässe Mittel geeignete Aktivatoren haben sich die in der EP-A 14 929 beschriebenen und beanspruchten Verbindungen erwiesen.

Die Aktivatoren werden in Konzentrationen von 0,5–10%, vorzugsweise 1–5% (w/v), in der Imprägnierlösung eingesetzt.

Zur Herstellung des erfindungsgemässen Mittels werden z.B. saugfähige Träger, vorzugsweise Filterpapier, Cellulose oder Kunstfaservliese, mit Lösungen der erforderlichen, üblicherweise zur Herstellung von Teststreifen verwendeten Reagenzien (Substrat, Puffer, gegebenenfalls Netzmittel) in leichtflüchtigen Lösungsmitteln, wie z.B. Wasser, Methanol, Ethanol oder Aceton, imprägniert. Dies geschieht zweckmässig in zwei getrennten Schritten: Zunächst wird mit einer wässrigen Lösung imprägniert, die den Puffer und andere wasserlösliche Zustzstoffe enthält. Danach wird mit einer Lösung der Protease-Substrate der allgemeinen Formel II bzw. III, Diazoniumsalzen der allgemeinen Formel I und Aktivatoren imprägniert. Die Imprägnierung kann jedoch auch in einer anderen Reihenfolge bzw. mit einer anderen Zusammensetzung der beiden Imprägnierlösungen durchgeführt werden.

Die fertigen Testpapiere können als solche verwendet werden oder in an sich bekannter Weise an Griffen angeklebt oder vorzugsweise zwischen Kunststoffen und feinmaschigen Netzwerken gemäss DE-C-2 118 455 eingesiegelt werden.

Zur Herstellung filmbeschichteter Teststreifen werden sämtliche Reagenzien in die Lösung oder Dispersion einer filmbildenden Substanz, wie z.B. Polyvinylester oder Polyamid, eingetragen und homogen vermischt. Das Gemisch wird in dünner Schicht auf einen Kunststoffträger gestrichen und getrocknet. Die erfindungsgemässen filmbeschichteten Teststreifen werden nach dem Trocknen geschnitten und können als solche verwendet werden oder in an sich bekannter Weise an Griffen angeklebt werden oder z.B. zwischen Kunststoffen und feinmaschigen Netzwerken gemäss DE-C-2 118 455 eingesiegelt werden.

Das erfindungsgemässe diagnostische Mittel zum Nachweis proteolytischer Enzyme, insbesondere der Leukozyten-Proteasen, in Form von Pulvermischungen oder Reagenztabletten lässt sich herstellen, indem die oben angeführten Bestandteile des Testes mit üblichen galenischen Zusatzstoffen versetzt und granuliert werden. Zusatzstoffe dieser Art sind z.B. Kohlenhydrate, wie z.B. Mono-, Oligo- oder Polysaccharide, oder Zuckeralkohole, wie z.B. Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 50–200 mg, vorzugsweise 50–80 mg, auf.

Zur Herstellung von Lyophilisaten im Gesamtgewicht von jeweils etwa 5–20 mg, vorzugsweise etwa 10 mg, wird eine Lösung gefriergetrocknet, die neben sämtlichen für den Test benötigten Reagenzien übliche Gerüstbildner, wie z.B. Polyvinylpyrrolidon, und evtl. weitere Füllstoffe, wie z.B. Mannit, Sorbit oder Xylit, enthält.

Das erfindungsgemässe diagnostische Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z.B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden. Die so hergestellten diagnostischen Mittel ermöglichen es, nach Eintauchen in die zu untersuchende oder nach Zugabe zur betreffenden Körperflüssigkeit die Anwesenheit proteolytischer Enzyme, insbesondere der Leukozyten-Proteasen, rasch und einfach über eine Farbbildung nachzuweisen, die visuell oder photometrisch, z.B. remissionsphotometrisch oder in der Küvette, beurteilt werden kann. Da die Aktivität der Leukozyten-Proteasen pro Zelle als eine im wesentlichen konstante Grösse angesehen werden kann, lässt sich aus der Intensität der Farbbildung die Leukozytenkonzentration der untersuchten Körperflüssigkeit ermitteln. Dabei werden mit dem erfindungsgemässen diagnostischen Mittel sowohl intakte als auch lysierte Leukozyten erfasst, da die Aktivität der Leukozyten-Proteasen auch nach der Lyse der Leukozyten voll erhalten bleibt. Ein Lysefehler tritt folglich nicht auf.

Beispiel 1

Filterpapier (z.B. Schleicher & Schüll 23 SL) wird nacheinander mit den folgenden Lösungen imprägniert und dann bei 60°C getrocknet:

Lösung 1
0,2 molarer Borat-Salzsäure-Puffer pH 8
10% Phosphorsäuretrimorpholid

Lösung 2
$2 \times 10^{-3}$ mol/l Substrat
$10^{-2}$ Diazoniumsalz

gelöst in Aceton/1-Decanol 98:2

Substrate
S 1:3-[N-(Toluol-4′-sulfonyl)-L-alanyloxy]-indol
S 2:3-[N-(Toluol-4′-sulfonyl)-L-alanyloxy]-1-methoxy-naphthalin
S 3:1-[N-(Toluol-4′-sulfonyl)-L-alanyloxy]-4-methoxy-naphthalin
S 4:1-[N-(Toluol-4′-sulfonyl)-L-alanyloxy]-4-isopropoxy-naphthalin
S 5:1-[N-(Toluol-4′-sulfonyl)-L-alanyloxy]-4-pentoxy-naphthalin
S 6:1-[N-(Toluol-4′-sulfonyl)-L-alanyloxy]-3-dimethylamino-benzol
S 7:1-[N-(Toluol-4′-sulfonyl)-L-alanyloxy]-3-diethylamino-benzol
S 8:1-[N-(Toluol-4′-sulfonyl)-L-alanyloxy]-3,5-dimethoxy-benzol
S 9:1-[N-(Toluol-4′-sulfonyl)-L-alanyloxy]-3-methyl-5-methoxy-benzol

Diazoniumsalze
D 1:2,4-Dimethoxy-benzoldiazonium-tetrafluoroborat
D 2:4-Methoxy-naphthalin-1-diazonium-tetrafluoroborat
D 3:2,5-Dimethoxy-4-dimethylamino-benzoldiazonium-tetrafluoroborat
D 4:4-Dimethylamino-benzoldiazonium-tetrafluoroborat

Man erhält Papiere, welche beim Eintauchen in leukozytenhaltige Harne 100 Leukozyten/µl in ca. 3 Minuten mit den in der Tabelle aufgeführten Farben anzeigen. Die Beurteilung kann auch remissionsphotometrisch erfolgen.

| Substrat | Diazonium-salz | Farbe |
|---|---|---|
| S1 | D1 | rot-braun |
| S1 | D2 | rot-braun |
| S1 | D3 | blau-grau |
| S1 | D4 | grün |
| S2 | D1 | rot |
| S2 | D2 | hellrot |
| S2 | D3 | violett |
| S2 | D4 | lila |
| S3 | D1 | rot-violett |
| S3 | D2 | violett |
| S3 | D3 | blau-grau |
| S4 | D1 | lila |
| S5 | D1 | lila |
| S6 | D1 | hellrot |
| S7 | D1 | hellrot |
| S8 | D1 | rot-violett |
| S9 | D1 | rot-braun |

Beispiel 2

Filterpapier wird nacheinander mit den folgenden Lösungen imprägniert und bei 60°C getrocknet.

Lösung 1
0,1 molarer Borat-Puffer pH 8

Lösung 2
$2\times10^{-3}$ mol/l 2-Methoxy-4-(N-morpholino)-benzoldiazoniumtetrachlorozinkat
$2\times10^{-3}$ mol/l Substrat*
10% Phosphorsäuretrimorpholid
in Ethanol/1-Decanol 98:2

*Substrat
A: 3-[N-Benzyloxycarbonyl-L-alanyloxy]-indol
B: 3-[N-Benzyloxycarbonyl-L-alanyloxy]-5-methylindol
C: 3-[N-Benzyloxycarbonyl-L-alanyloxy]-7-methylindol
D: 3-[N-Benzyloxycarbonyl-L-alanyloxy]-4-chlorindol
E: 3-[N-Benzyloxycarbonyl-L-alanyloxy]-5-bromindol
F: 3-[N-(Toluol-4′-sulfonyl)-L-alanyloxy]-indol
G: 3-[N-(Toluol-4′-sulfonyl)-L-alanyloxy]-5-methoxy-indol
H: 3-[N-(4′-acetamidobenzolsulfonyl)-L-alanyloxy]-indol
I: 3-[N-(4-methoxytosyl)-L-alanyloxy]-indol
K: [N-(Toluol-4′-sulfonyl)-L-alanyloxy]-benzol
L: 1-[N-(Toluol-4′-sulfonyl)-L-alanyoxy]-naphthalin
M: 1-[N-(Toluol-4′-sulfonyl)-L-alanyloxy]-3-methoxy-benzol

Mit den so erhaltenen Reagenzpapieren lassen sich in leukozytenhaltigen Harnen 100 Leukozyten/µl nachweisen.

Reagenzpapiere mit vergleichbarer Reaktivität lassen sich auch erhalten, wenn man anstelle des oben genannten Diazoniumsalzes
2-Methoxy-4-(N-pyrrolidino)-benzoldiazonium-tetrafluoroborat
2-Methoxy-4-(N-piperidino)-benzoldiazonium-tetrafluoroborat
2,6-Di-methoxy-4(N-morpholino)-benzoldiazonium-tetrafluoroborat
4-Methoxy-2-(N-morpholino)-benzoldiazonium-tetrafluoroborat
2-Methoxy-4-[N(N′-methyl)-piperazino]-benzoldiazoniumtetrafluoroborat
2-Methoxy-4-(N-thiomorpholino)-benzoldiazonium-tetrafluoroborat

Beispiel 3

Es werden folgende Stammlösungen hergestellt:

Lösung 1
$2\times10^{-3}$ mol/l 1-[N-(Toluol-4′-sulfonyl)-L-alanyloxyl]-4-isopropoxy-naphthalin
$10^{-2}$ mol/l 2,4-Dimethoxy-benzoldiazonium-tetrafluoroborat
in Aceton/1-Decanol 98:2

Lösung 2
0,2 molarer Borax-Salzsäure-Puffer
10% Phosphorsäuretrimorpholid

Nachdem jeweils entweder zu Lösung 1 oder zu Lösung 2 die unten aufgeführten Aktivatoren in den dort angegebenen Konzentrationen zugefügt worden sind, wurden Filterpapiere mit den Lösungen 1 und 2 getränkt und jeweils bei 60°C getrocknet.

Bei der Überprüfung in isotonischer Kochsalzlösung mit 300 Leukozyten/µl reagierten die Teste in den angegebenen Zeiten.

| Aktivator | mg Aktivatorzusatz zu 20 ml von Lösung 1 | Lösung 2 | Reaktionszeit [sec] |
|---|---|---|---|
| ohne | – | – | 54 |
| 4-Azafluoren | 33 | - | 17 |
| Benzo-(h)-chinolin | 36 | - | 8 |
| Chinin | 33 | - | 32 |
| 1,2-Bis-4-pyridyl-ethylen | 37 | - | 34 |
| 1-Decanol | 400 | - | 5 |
| 1-Tetradecanol | 400 | - | 4 |
| Nitroprussid-natrium | - | 31 | 38 |
| Kaliumhexacyanoferrat-(II) | - | 40 | 30 |

Beispiel 4

Filterpapier wird mit den folgenden Lösungen getränkt und nach dem Tränken jeweils bei 60°C getrocknet.

Lösung 1
$2\times10^{-3}$ mol/l 3-(N-Toluol-4′-sulfonyl-L-alanyl-oxy)-indol
$2\times10^{-3}$ mol/l 2-Methoxy-4-morpholino-benzoldiazoniumtetrachlorozinkat
in Aceton/1-Decanol 98:2

Lösung 2
0,2 molarer Borax-Salzsäure-Puffer pH 8
10% Phosphorsäuretrimorpholid

In einem weiteren Versuch wurde in Lösung 2 Phosphorsäuretrimorpholid weggelassen.

Die Muster mit Phosphorsäuretrimorpholid blieben nach einer Belastung mit 60°C über 2 Tage unverfärbt und lieferten mit leukozytenhaltigen Lösungen gute Reaktionen. Die Muster ohne Phosphorsäuretrimorpholid zeigen graue Verfärbungen.

Beispiel 5

In bekannter Weise wurde eine Reagenztablette hergestellt, die folgende Komponenten enthielt:
2 mg 3-(N-Toluol-4′-sulfonyl-L-alanyloxy)-indol
2 mg 2-Methoxy-4-(N-morpholino)-benzol-diazoniumtetrachlorzinkat
1,5 mg Kaliumdihydrogenphosphat
30 mg Dinatrium-hydrogenphosphat-dihydrat
20 mg Mannit

Diese Tablette wurde in 2 ml leukozytenhaltigen Harn aufgelöst und gut durchmischt. Bei Anwesenheit von Leukozyten trat eine Rotviolettfärbung auf.

100 Leukozyten konnten so in ca. 2 Minuten nachgewiesen werden.

Wurde der Urin vor der Tablettenzugabe und während der Reaktion auf 37°C temperiert, so konnten in der gleichen Zeit 20 Leukozyten nachgewiesen werden.

Beispiel 6

1-[N-(Toluol-4′-sulfonyl)-L-alanyloxy]-3,5-dimethoxy-benzol

Zur Umsetzung nach dem Aktivester-Verfahren werden 14,6 g (0,06 Mol) N-(Toluol-4-sulfonyl)-L-alanin und 12,2 g (0,09 Mol) N-Hydroxybenzotriazol in 300 ml abs. Essigester gelöst, auf 0°C abgekühlt und mit 12,4 g (0,06 Mol) Dicyclohexylcarbodiimid versetzt. Zur Bildung des Aktivesters rührt man 2 Stunden bei 0°C, dann weitere 2 Stunden bei Raumtemperatur. Nach Zugabe von 6.2 g (0,04 Mol) 3.5-Dimethoxyphenol und 5.5 ml (0,04 Mol) Triethylamin wird das Gemisch 15 Stunden bei Raumtemperatur gerührt. Der gebildete N,N′-Dicyclohexylharnstoff wird abgesaugt. Das Filtrat wird im Vakuum bei maximal 50°C Badtemperatur eingeengt. Der Rückstand wird in 200 ml Essigester aufgenommen und nacheinander je dreimal mit je 100 ml 5%-iger Zitronensäure und dann entsprechend mit je 100 ml 5%-iger Natriumhydrogencarbonatlösung gewaschen. Die organische Phase wird nach Trocknen mit Natriumsulfat im Vakuum eingedampft. Das ölige Rohprodukt wird säulenchromatographisch an einer Kieselgelsäure mit einem Toluol-Essigester-Gemisch (4:1) gereinigt. Nach Einengen der entsprechenden gesammelten Fraktionen wird der Rückstand in wenig Methylenchlorid gelöst und durch Zugabe von Ether ausgefällt. Es ergeben sich
5.8 g (38%) 1-[N-(Toluol-4′-solfonyl)-L-alanyloxy]-3,5-dimethoxy-benzol, farblose Kristalle, Schmp. 110°C

$\alpha_D^{20}$: -52.5° (c = 1%, Aceton)

In analoger Weise erhält man durch Reaktion von N-(Toluol-4-sulfonyl)-L-alanin mit den entsprechend substituierten Phenolen oder Naphtholen die folgenden Substanzen:

6.1 [N-(Toluol-4-sulfonyl)-L-alanyloxy]-benzol, farblose Kristalle, Schmp. 113°C

$\alpha_D^{20}$: -63.8° (c = 1%, Aceton)

6.2 1-[N-(Toluol-4′-sulfonyl)-L-alanyloxy]-3-dimethylamino-benzol
farbloses, amorphes Pulver

$\alpha_D^{20}$: -36.6° (c = 1%, Methanol)

DC: Fertigplatte Kieselgel
(Laufmittel: Toluol-Dioxan 2:1, Detektion: UV,
$R_F$-Wert: 0,68)

6.3 1-[N-(Toluol-4′-sulfonyl)-L-alanyloxy]-3-methoxybenzol
farblose Kristalle, Schmp. 60–61°C

$\alpha_D^{20}$: -62.9° (c = 1%, Methanol)

6.4 1-[N-(Toluol-4′-sulfonyl)-L-alanyloxy]-naphthalin
farbloses viskoses Öl

$\alpha_D^{20}$: -27.3° (c = 1%, Methanol)

DC: Fertigplatte Kieselgel
(Laufmittel: Toluol-Dioxan 4:1, Detektion: UV,
$R_F$-Wert: 0,53)

6.5 1-[N-(Toluol-4′-sulfonyl)-L-alanyloxy]-4-methoxynaphthalin
farblose Kristalle, Schmp. 136°C

$\alpha_D^{20}$: -42.6° (c = 1%, Aceton)

6.6 1-[N-(Toluol-4′-sulfonyl)-L-alanyloxy]-4-isopropoxynaphthalin
farblose Kristalle, Schmp. 93–96°C

$\alpha_D^{20}$: -38.0° (c = 1%, Aceton)

6.7 1-[N-(Toluol-4′-sulfonyl)-L-alanyloxy]-4-pentoxynaphthalin
farblose Kristalle, Schmp. 87°C

$\alpha_D^{20}$: -36,9° (c = 1%, Aceton)

6.8 1-[N-(Toluol-4′-sulfonyl)-L-alanyloxy]-3-diethylamino-benzol
farblose Kristalle, Schmp. 83–84°C

$\alpha_D^{20}$: -59,4° (c = 1%, Aceton)

DC: Fertigplatte Kieselgel
(Laufmittel: Xylol-Methylethylketon 1:1
$R_F$-Wert: 0,68)

6.9 1-[N-(Toluol-4′-sulfonyl)-L-alanyloxy]-3-methyl-5-methoxy-benzol
farblose Kristalle, Schmp. 79–81°C

$\alpha_D^{20}$: 62,2° (c = 1%, Methanol)

DC: Fertigplatte Kieselgel
(Laufmittel: Chloroform-Methanol 20:1
$R_F$-Wert: 0,79)

Beispiel 7
2-[N-(Toluol-4′-sulfonyl)-L-alanyloxy]-4-methoxynaphthalin

Lösung 1

Zur Herstellung des Säurechlorids nach der Einstufen-Methode werden 19,44 g (0,08 Mol) N-(Toluol-4-sulfonyl)-L-alanin in 100 ml abs. Dimethylformamid gelöst und auf -20°C abgekühlt. Dann werden unter Rühren und Kühlen 5,81 ml (0,08 Mol) Thionylchlorid zupipettiert und das Reaktionsgemisch 30 Minuten im Kältebad bei -20°C belassen.

Lösung 2

Zu einer Lösung von 6,96 g (0,04 Mol) 4-Methoxy-naphthol-2 in 50 ml abs. Dimethylformamid werden 11,0 ml (0,08 Mol) Triethylamin gegeben. Das Gemisch wird auf -20°C abgekühlt.

Umsetzung

Man giesst Lösung 1 zu Lösung 2 und rührt unter Wasserausschluss ca. 4 Stunden bei -20°C, danach belässt man das Gemisch über Nacht im Kühlschrank.

Zur Aufarbeitung wird die Reaktionslösung im Vakuum bei maximal 50°C Badtemperatur eingeengt. Der Rückstand wird in ca. 150 ml Essigester aufgenommen und nacheinander je dreimal mit je 100 ml 5%-iger Zitronensäure und dann mit je 100 ml 5%-iger Natriumhydrogencarbonatlösung gewaschen. Nach Trocknen mit Natriumsulfat wird die organische Phase im Vakuum eingedampft. Das Rohprodukt wird säulenchromatographisch an einer Kieselgelsäule mit einem Toluol-Essigester-Gemisch (4:1) gereinigt. Nach Einengen der entsprechenden gesammelten Fraktionen im Vakuum wird der Rückstand in wenig Methylenchlorid gelöst und dann mit einem Ether-Ligroin-Gemisch (1:2) gefällt. Es werden erhalten

2.8 g (18%) 2-[N-(Toluol-4′-sulfonyl)-L-alanyloxy]-4-methoxy-naphthalin
farblose Kristalle, Schmp. 119°C

$\alpha_D^{20}$: -57.9° (c = 1%, Aceton)

Beispiel 8

2-Methoxy-4-(N-morpholino)-benzoldiazoniumtetrachlorozinkat

5-Chor-2-nitroanisol wird mit 1,5-fachem molarem Überschuss an Morpholin in Toluol als Lösungsmittel durch mehrstündiges Erhitzen am Rückfluss (10–14 Stunden) umsetzt. Die Umsetzung kann auch zweckmässigerweise in Morpholin als Lösungsmittel durchgeführt werden. Hierzu wird das 5-Chlor-2-nitroanisol mit dem 5- bis 10-fachen Volumen an Morpholin versetzt. Eventuell entmethyliertes Produkt wird durch Schütteln mit Natronlauge abgetrennt oder durch Umsetzen mit Diazomethan nachmethyliert.

Die erhaltene Nitroverbindung wird in üblicher Weise mit Palladium/Kohle in Methanol oder Zinn-(II)-chlorid in Salzsäure zum Amid reduziert und dieses diazotiert. Die Diazoniumverbindung wird in bekannter Weise in salzsaurer Lösung durch Zugabe konzentrierter Zinkchloridlösung in das Tetrachlorozinkat oder durch Zugabe von Tetrafluoroborsäure in das Tetrafluoroborat überführt und als solches isoliert.

Fp. 170–172°C (Tetrachlorozinkat)
166–168°C (Tetrafluoroborat)

Beispiel 9

4-Methoxy-2-(N-morpholino)benzoldiazonium-tetrafluoroborat

18,76 g (0,1 Mol) 3-Chlor-4-nitroanisol, Fp. 52°C, werden mit 87,1 g (1 Mol) Morpholin 4 Stunden unter Rückfluss erhitzt. Danach kühlt man auf Raumtemperatur, gibt zur Reaktionslösung 100 ml Eiswasser, saugt das abgeschiedene, gelbe Reaktionsprodukt ab, wäscht mit eisgekühlter 10%iger Essigsäure und trocknet das erhaltene Material bei 60°C im Vakuum. Es resultieren 19,5 g Gemisch aus N-(3-Hydroxy-6-nitrophenyl)-morpholin und N(3-Methoxy-6-nitrophenyl)-morpholin. Man löst dieses Produkt in Methylenchlorid und gibt eine etherische Lösung von Diazomethan zu. Nach zweitägigem Stehen bei Raumtemperatur wird das überschüssige Diazomethan durch Zutropfen von 2N Essigsäure zerstört, die organische Phase mehrmals mit 2N Natronlauge durchgeschüttelt und das Lösungsmittel im Vakuum abgedampft. Man erhält 19,1 g (= 80,2%) N-(3-Methoxy-6-nitrophenyl)morpholin, Fp. 84–86°C. Anschliessend wird diese Substanz in 250 ml Methanol suspendiert und nach Zugabe von 1,9 g Palladium-Kohle (10%ig) bei 20–30°C hydriert.

Nach Absaugen vom Katalysator, engt man weitgehend ein und führt die freigesetzte Base durch Zugabe von etherischer Salzsäure in das Hydrochlorid über. Es kristallisieren 20,6 g (= 73,1% d.Th.) N(3-Methoxy-6-amino-phenyl)morpholin-dihydrochlorid, Fp. 237–240°C.

Man suspendiert dieses Material bei -5°C in 96 ml 6N Salzsäure und tropft innerhalb 30 Minuten eine Lösung von 6 g Natriumnitrit in 12 ml Wasser zu; die entstandene braunrote Diazoniumsalzlösung gibt man bei 0°C unter Rühren zu 120 ml 35%iger Tetrafluoroborsäure. Nach mehrstündigem Stehen erhält man 19,5 g (= 63,5% d.Th.) 4-Methoxy-2-(N-morpholino)-benzoldiazonium-tetrafluoroborat, gelbe Kristalle, Fp. 115–116°C (Zers.)

In analoger Weise erhält man

a) aus 5-Chlor-2-nitroanisol und Thiomorpholin das 2-Methoxy-4-(N-thiomorpholino)-benzoldiazonium-tetrafluoroborat
Fp 106–108°C (Zers.)

Das Zwischenprodukt 2-Amino-5(N-thiomorpholino)-anisoldihydrochlorid wird wie folgt hergestellt:

In einem 1l-Dreihalskolben mit Rührer trägt man 100 g Zinn-II-chloriddihydrat in 400 ml 6N Salzsäure ein und fügt 25,4 g (1 Mol) 2-Nitro-5-(N-thiomorpholino)anisol portionsweise bei Raumtemperatur unter Rühren zu. Danach erwärmt man 30 Minuten auf 75°C, kühlt auf Raumtemperatur und tropft die Reaktionslösung unter Eiskühlung in 750 ml 30%ige Natronlauge ein. Man extrahiert die freigesetzte Base mehrmals mit Ether und führt diese nach Trocknen und teilweisem Verdampfen des Lösungsmittels durch Zugabe von etherischer Salzsäure in das Hydrochlorid über. Man erhält 24,9 g (= 83,8% d.Th.) 2-Amino-5(N-thiomorpholino)-anisoldihydrochlorid. Fp. 218–220°C

b) aus 5-Chlor-2-nitroanisol und Piperidin das 2-Methoxy-4-(N-piperidino)-benzoldiazonium-tetrafluoroborat
Fp. 123–125°C (Zers.)

c) aus 5-Chlor-2-nitroanisol und Pyrrolidin das 2-Methoxy-4-(N-pyrrolidino)-benzoldiazonium-tetrafluoroborat
Fp. 137–139°C (Zers.)

d) aus 5-Chlor-2-nitroanisol und N-Methylpiperazin das 2-Methoxy-4-[N(N′-methyl)-piperazino]-benzoldiazonium-tetrafluoroborat-dihydro-tetrafluoroborat
Fp. 163°C (Zers.)

Bei der Herstellung dieser Verbindung wird die Diazotierung mit Amylnitrit in Methanol durchgeführt. Hierzu werden 39,6 g (0,1 Mol) 2-Amino-5-(N-methyl-piperazino)anisol-dihydro-tetrafluoroborat in 175 ml Methanol gelöst, 11,6 g (0,1 Mol) Amylnitrit in 25 ml Methanol zugegeben und langsam unter Rühren bei 0°C 60 ml 35%ige Tetrafluorborsäure zugetropft. Beim Stehen kristallisieren 24,8 g (= 51,7% d.Th.) bräunliche Kristalle von 2-Methoxy-4[N-(N′-methyl)piperazino]-benzoldiazonium-tetrafluoroborat-dihydro-tetrafluoroborat
Fp. 163°C (Zers.)

e) aus 5-Chlor-2-nitroanisol und Morpholin das 2-Methoxy-4-(N-morpholino)-benzoldiazoniumtetrafluoroborat)
Fp. 166–168°C (Zers.)

**Patentansprüche**

1. Verwendung von Mitteln zum Nachweis esterolytischer und/oder proteolytischer Enzyme, bestehend aus einem saugfähigen Träger, einer Filmschicht, einer Pulvermischung, einem Lyophilisat, einer Lösung oder einer Reagenztablette, enthaltend ein geeignetes Esterase- und/

oder Protease-Nachweisprinzip, einen Puffer sowie gegebenenfalls weitere, üblicherweise verwendete Zusatzstoffe zum Nachweis von Leukozyten in Urin, wobei das Mittel dem zu untersuchenden Urin zugegeben wird und die entstehende Farbbildung visuell oder photometrisch beurteilt wird, dadurch gekennzeichnet, dass als Esterase- und/oder Protease-Nachweisprinzip eine Kombination aus einem speziell substituierten Diazoniumsalz und einem geeigneten Ester eingesetzt wird, deren Reaktivität durch geeignete Auswahl der Substituenten derart aufeinander abgestimmt wird, dass zwar eine hinreichend rasche Kupplung mit der aus dem Ester freigesetzten Alkohol-Komponente, jedoch keine Reaktion mit sonstigen Bestandteilen der Testlösung erfolgt, wobei der Ester aus der Gruppe der Verbindungen der allgemeinen Formel II

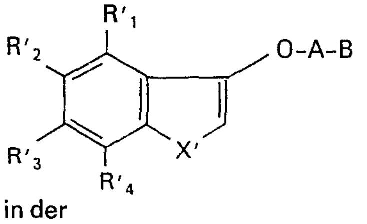

(II),

in der

$R'_1$, $R'_2$, $R'_3$, $R'_4$, die jeweils gleich oder verschieden sein können, jeweils ein Wasserstoff- oder ein Halogenatom, eine niedere Alkyl-, eine niedere Alkoxy-, eine Aryl-, eine Aralkyl-, eine Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylamino-Gruppe oder jeweils zwei benachbarte Substituenten einen gegebenenfalls durch Halogen substituierten benzoannellierten Rest,

X′ ein Schwefelatom oder eine gegebenenfalls durch einen niederen Alkyl-, einen Aryl-, einen Aralkyl- oder einen Acylrest substituierte Iminogruppe,

A einen Aminosäure- oder einen Peptidrest,

B eine in der Peptidchemie übliche oder davon abgeleitete Stickstoffschutzgruppe

bedeuten,

oder der Verbindungen der allgemeinen Formel III

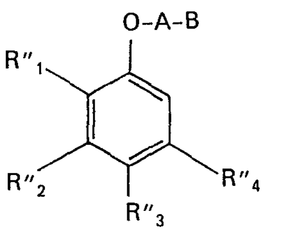

(III),

in der

$R''_1$, $R''_2$, $R''_3$, $R''_4$, die gleich oder verschieden sein können, jeweils Wasserstoff, eine niedere Alkyl- eine niedere Alkoxy-, Alkylamino- oder Dialkylamino-Gruppe bedeuten, wobei zwei benachbarte Substituenten auch einen annellierten Benzolring vorstellen können,

A und B die oben angegebene Bedeutung haben, ausgewählt wird.

2. Verwendung von Mitteln gemäss Anspruch 1, dadurch gekennzeichnet, dass das Esterase- und/oder Protease-Nachweisprinzip als Diazonium-Salz eine Verbindung der allgemeinen Formel I

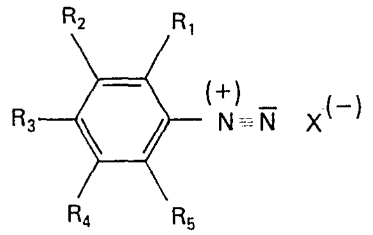

(I),

in der

$R_1$ eine niedere Alkyl-, eine niedere Alkoxy-, eine niedere Alkylmercapto-, eine N-Morpholino-, eine N-Thio-morpholino-, eine N-Pyrrolidino-, eine gegebenenfalls N′-alkylierte N-Piperazino-, eine N-Piperidino-Gruppe, Halogen oder Wasserstoff,

$R_3$ eine niedere Alkyl-, eine niedere Alkoxy-, eine Aryloxy-, eine niedere Alkylmercapto-, Alkylamino-, Dialkylamino-, eine Hydroxy-, eine N-Morpholino-, N-Thio-morpholino-, N-Pyrrolidino-, eine gegebenenfalls N′-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasserstoff,

$R_2$, $R_4$, $R_5$, die gleich oder verschieden sein können, jeweils eine niedere Alkyl-, eine niedere Alkoxy-, eine niedere Alkylmercapto-Gruppe, Halogen oder Wasserstoff, wobei $R_1$ und $R_2$ zusammen einen annellierten Benzolring vorstellen können, und

X ein stabilisierendes Anion

bedeuten, enthält.

3. Verwendung gemäss Anspruch 1, dadurch gekennzeichnet, dass als zusätzliche Hilfsmittel Netzmittel, Stabilisatoren, Aktivatoren, Filmbildner, galenische Zusatzstoffe und/oder Gerüstbildner verwendet werden.

4. Mittel zum Nachweis esterolytischer und/oder proteolytischer Enzyme in Leukozyten im Urin, bestehend aus einem saugfähigen Träger, einer Filmschicht, einer Pulvermischung, einem Lyophilisat, einer Lösung oder einer Reagenztablette, enthaltend ein geeignetes Esterase- und/oder Protease-Nachweisprinzip, einen Puffer sowie gegebenenfalls weitere, üblicherweise verwendete Zusatzstoffe, dadurch gekennzeichnet, dass als Esterase- und/oder Protease-Nachweisprinzip eine Kombination aus einem speziell substituierten Diazoniumsalz und einem geeigneten Ester eingesetzt wird, deren Reaktivität durch geeignete Auswahl der Substituenten derart aufeinander abgestimmt wird, dass zwar eine hinreichend rasche Kupplung mit der aus dem Ester freigesetzten Alkohol-Komponenten, jedoch keine Reaktion mit sonstigen Bestandteilen der Testlösung erfolgt, wobei der Ester aus der Gruppe der Verbindungen der allgemeinen Formel II

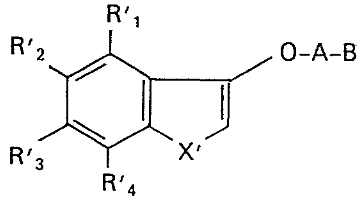

(II),

in der

R'$_1$, R'$_2$, R'$_3$, R'$_4$, die jeweils gleich oder verschieden sein können, jeweils ein Wasserstoff- oder ein Halogenatom, eine niedere Alkyl-, eine niedere Alkoxy-, eine Aryl-, eine Aralkyl-, eine Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylamino-Gruppe oder jeweils zwei benachbarte Substituenten einen gegebenenfalls durch Halogen substituierten benzoannellierten Rest,

X' ein Schwefelatom oder eine gegebenenfalls durch einen niederen Alkyl-, einen Aryl-, einen Aralkyl- oder einen Acylrest substituierte Iminogruppe,

A einen Aminosäure- oder einen Peptidrest,

B eine in der Peptidchemie übliche oder davon abgeleitete Stickstoffschutzgruppe

bedeuten,

oder der Verbindungen der allgemeinen Formel III

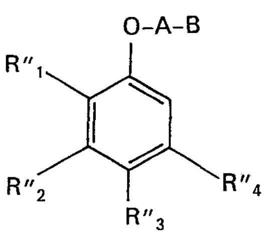

(III),

in der

R''$_1$, R''$_2$, R''$_3$, R''$_4$, die gleich oder verschieden sein können, jeweils Wasserstoff, eine niedere Alkyl-, eine niedere Alkoxy-, Alkylamino- oder Dialkylamino-Gruppe bedeuten, wobei zwei benachbarte Substituenten auch einen annellierten Benzolring vorstellen können,

A und B die oben angegebene Bedeutung haben, wobei wenn R''$_1$ und R''$_2$ oder R''$_2$ und R''$_3$ ein annellierter Benzolring ist, B nicht eine Acetyl- oder Benzyloxycarbonylgruppe darstellt,

ausgewählt wird.

5. Mittel gemäss Anspruch 4, dadurch gekennzeichnet, dass als Esterase- und/oder Protease-Nachweisprinzip eine Kombination aus einem Diazonium-Salz der allgemeinen Formel I

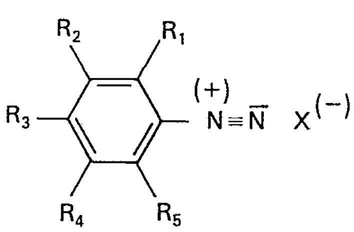

(I),

in der

R$_1$ eine niedere Alkyl-, eine niedere Alkoxy-, eine niedere Alkylmercapto-, eine N-Morpholino-, eine N-Thio-morpholino-, eine N-Pyrrolidino-, eine gegebenenfalls N'-alkylierte N-Piperazino-, eine N-Piperidino-Gruppe, Halogen oder Wasserstoff,

R$_3$ ein niedere Alkyl-, eine niedere Alkoxy-, eine Aryloxy-, eine niedere Alkylmercapto-, Alkylamino-, Dialkylamino-, eine Hydroxy-, eine N-Morpholino-, N-Thio-morpholino-, N-Pyrrolidino-, eine gegebenenfalls N'-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasserstoff,

R$_2$, R$_4$, R$_5$, die gleich oder verschieden sein können, jeweils eine niedere Alkyl-, eine niedere Alkoxy-, eine niedere Alkylmercapto-Gruppe, Halogen oder Wasserstoff, wobei R$_1$ und R$_2$ zusammen einen annellierten Benzolring vorstellen können, und

X ein stabilisierendes Anion

bedeuten, und einem Ester, ausgewählt aus der Gruppe der Verbindungen der allgemeinen Formel II

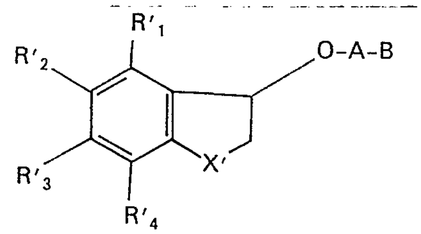

(II),

in der

R'$_1$, R'$_2$, R'$_3$, R'$_4$, die jeweils gleich oder verschieden sein können, jeweils ein Wasserstoff- oder ein Halogenatom, eine niedere Alkyl-, eine niedere Alkoxy-, eine Aryl-, eine Aralkyl-, eine Aralkoxy- eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylamino-Gruppe oder jeweils zwei benachbarte Substituenten einen gegebenenfalls durch Halogen substituierten benzoannellierten Rest,

X' ein Schwefelatom oder eine gegebenenfalls durch einen niederen Alkyl-, einen Aryl-, einen Aralkyl- oder einen Acylrest substituierte Iminogruppe,

A einen Aminosäure- oder einen Peptidrest,

B eine in der Peptidchemie übliche oder davon abgeleitete Stickstoffschutzgruppe

bedeuten, oder der Verbindungen der allgemeinen Formel III

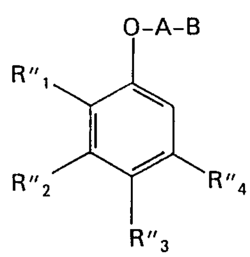

(III),

in der
$R''_1$, $R''_2$, $R''_3$, $R''_4$, die gleich oder verschieden sein können, jeweils Wasserstoff, eine niedere Alkyl-, eine niedere Alkoxy-, Alkylamino- oder Dialkylamino-Gruppe bedeuten, wobei zwei benachbarte Substituenten auch einen annellierten Benzolring vorstellen können,
A und B die oben angegebene Bedeutung haben, wobei wenn $R''_1$ und $R''_2$ oder $R''_2$ und $R''_3$ ein annellierter Benzolring ist, B nicht eine Acetyl- oder Benzyloxycarbonylgruppe darstellt,
eingesetzt werden.

6. Mittel gemäss Anspruch 4, dadurch gekennzeichnet, dass als zusätzliche Hilfsmittel Netzmittel, Stabilisatoren, Aktivatoren, Filmbildner, galenische Zusatzstoffe und/oder Gerüstbildner verwendet werden.

7. Phenoxy-Aminosäureester und -Peptidester der allgemeinen Formel III

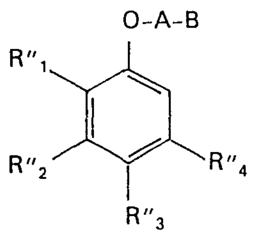
(III),

in der
$R''_1$, $R''_2$, $R''_3$, $R''_4$, die gleich oder verschieden sein können, jeweils Wasserstoff, eine niedere Alkyl-, eine niedere Alkoxy-, Alkylamino- oder Dialkylamino-Gruppe bedeuten, wobei zwei benachbarte Substituenten auch einen annellierten Benzolring vorstellen können,
A und B die oben angegebene Bedeutung haben, wobei wenn $R''_1$ und $R''_2$ bzw. $R''_2$ und $R''_3$ ein annellierter Benzolring ist, B nicht eine Acetyl- oder Benzyloxycarbonylgruppe darstellt.

8. Verfahren zur Herstellung von Phenoxy-Aminosäureestern und -Peptidestern gemäss Anspruch 7,
dadurch gekennzeichnet, dass man Verbindungen der allgemeinen Formel IV

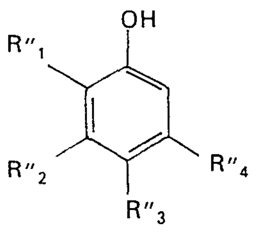
(IV),

in der
$R''_1$, $R''_2$, $R''_3$ und $R''_4$ die oben angegebene Bedeutung haben,
mit Aminosäuren und Peptiden der allgemeinen Formel V

HO-A-B (V),

in der
A und B die oben angegebene Bedeutung haben, bzw. geeigneten reaktiven Derivaten hiervon nach in der Peptidchemie üblichen Methoden umsetzt.

9. Verwendung von Verbindungen gemäss Anspruch 7 zur Herstellung von Mitteln gemäss Anspruch 4.

## Revendications

1. Utilisation d'agents pour la mise en évidence d'enzymes estérolytiques et/ou protéolytiques, comprenant un support absorbant, une couche de film, un mélange pulvérulent, un produit lyophilisé, une solution ou un comprimé réactif, renfermant un mélange pour la mise en évidence d'estérase et/ou de protéase approprié, un tampon et éventuellement d'autres additifs habituellement utilisés pour la mise en évidence de leucocytes dans l'urine, l'agent étant ajouté à l'urine à examiner et la formation d'un colorant étant jugée visuellement ou par photométrie, caractérisé en ce que le mélange pour la mise en évidence d'estérase et/ou de protéase utilisé est une combinaison d'un sel de diazonium specialement substitué et d'un ester approprié, dont les réactivités ont été accordées entre elles par un choix judicieux des substituants tel qu'il se produise bien une copulation suffisamment rapide avec l'alcool libéré à partir de l'ester, mais qu'il ne se produise aucune réaction avec les autres ingrédients de la solution d'essai, l'ester étant choisi dans le groupe des composés de formule générale II

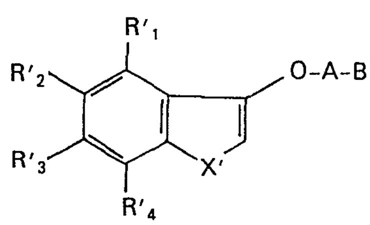
(II)

dans laquelle
$R'_1$, $R'_2$, $R'_3$, $R'_4$ pouvant être selon les cas identiques ou différents, sont chacun un atome d'hydrogène ou d'halogène, un groupe alkyle inférieur, alcoxy inférieur, aryle, aralkyle, aralcoxy, hydroxyle, carboxyle, carboxy-alcoxy inférieur, aralcoxycarbonyle, aralcoxycarbonyl-alcoxy inférieur, nitro ou acylamino inférieur, ou bien chaque fois deux substituants voisins forment un reste benzocyclique éventuellement substitué par de l'halogène,
X' est un atome de soufre ou un groupe imino éventuellement substitué par un reste alkyle inférieur, aryle, aralkyle ou acyle,
A est un reste d'un acide aminé ou d'un peptide
B est un groupe de protection de l'azote habituellement utilisé dans la chimie des peptides ou un dérivé d'un tel groupe
ou des composés de formule générale III

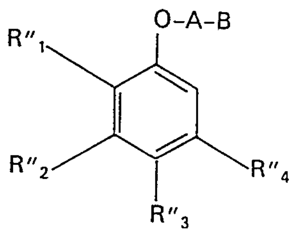

(III)

dans laquelle
$R''_1$, $R''_2$, $R''_3$, $R''_4$, pouvant être identiques ou différents, sont chacun de l'hydrogène, un groupe alkyle inférieur, alcoxy inférieur, alkylamino ou dialkylamino, deux substituants voisins pouvant également former un noyau benzocyclique,
A et B ont la signification indiquée ci-dessus.

2. Utilisation d'agents selon la revendication 1, caractérisée en ce que le mélange pour la mise en évidence d'estérase et/ou de protéase utilisé contient comme sel de diazonium un composé de formule générale I

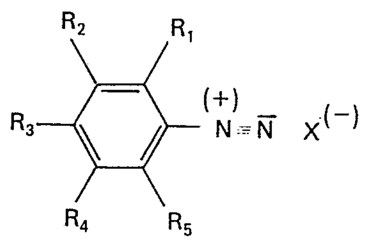

(I),

dans laquelle
$R_1$ est un groupe alkyle inférieur, alcoxy inférieur, alkylmercapto inférieure, N-morpholino, N-thiomorpholino, N-pyrrolidino, N-pipérazino éventuellement N'-alkylé, N-pipéridino, de l'halogène ou de l'hydrogène,
$R_3$ est un groupe alkyl inférieur, alcoxy inférieur, aryloxy, alkylmercapto inférieur, alkylamino, dialkylamino, hydroxyle, N-morpholino, N-thiomorpholino, N-pyrrolidino, N-pipérazino éventuellement N'-alkylé, N-pipéridino, phénylamino, phényle éventuellement substitué par un reste alkyle inférieur ou alcoxy inférieur, de l'halogène ou de l'hydrogène,
$R_2$, $R_4$, $R_5$ pouvant être identiques ou différents sont chacun un groupe alkyle inférieur, alcoxy inférieur, alkylmercapto inférieur, de l'halogène ou de l'hydrogène, $R_1$ et $R_2$ pouvant former ensemble un noyau benzocyclique et
X est un anion stabilisant.

3. Agent selon la revendication 1 caractérisé en ce qu'on utilise comme agents auxiliaires complémentaires des agents mouillants, des stabilisants, des activateurs, des agents filmogènes, des additifs galénques et/ou des formateurs de structures.

4. Agent pour la mise en évidence d'enzymes estérolytiques et/ou protéolytiques des leucocytes dans l'urine, comprenant un support absorbant, une couche de film, un mélange pulvérulent, un produit lyophilisé, une solution ou un comprimé réactif, renfermant un mélange pour la mise en évidence d'estérase et/ou de protéase approprié, un tampon et éventuellement d'autres additifs habituellement utilisés, caractérisé en ce que le mélange pour la mise en évidence d'estérase et/ou de protéase utilisé est une combinaison d'un sel de diazonium spécialement substitué et d'un ester approprié, dont les réactivités ont été accordées entre elles par un choix judicieux des substituants tel qu'il se produise bien une copulation suffisamment rapide avec l'alcool libéré à partir de l'ester, mais qu'il ne se produise aucune réaction avec les autres ingrédients de la solution d'essai, l'ester étant choisi dans le groupe des composés de formule générale II

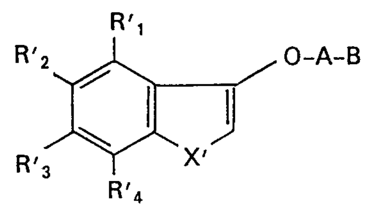

(II),

dans laquelle
$R'_1$, $R'_2$, $R'_3$, $R'_4$ pouvant être selon les cas identiques ou différents, sont chacun un atome d'hydrogène ou d'halogène, un groupe alkyle inférieur, alcoxy inférieur, aryle, aralkyle, aralcoxy, hydroxyle, carboxyle, carboxy-alcoxy inférieur, aralcoxycarbonyle, aralcoxy-carbonyl-alcoxy inférieur, nitro ou acylamino inférieur, ou bien chaque fois deux substituants voisins forment un reste benzocyclique éventuellement substitué par de l'halogène,
X' est un atome de soufre ou un groupe imino éventuellement substitué par un reste alkyle inférieur, aryle, aralkyle ou acyle,
A est un reste d'un acide aminé ou d'un peptide
B est un groupe de protection de l'azote habituellement utilisé dans la chimie des peptides ou un dérivé d'un tel groupe ou des composés de formule générale III

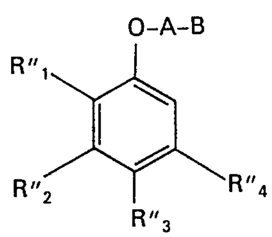

(III),

dans laquelle
$R''_1$, $R''_2$, $R''_3$, $R''_4$, pouvant être identiques ou différents, sont chacun de l'hydrogène, un groupe alkyle inférieur, alcoxy inférieur, alkylamino ou dialkylamino, deux substituants voisins pouvant également former un noyau benzocyclique,
A et B ont la signification indiquée ci-dessus à l'exaption de B ne pouvant être un group acétyl on benzyle-oxycarbonyle si $R''_1$ et $R''_2$ ou $R''_2$ et $R''_3$ forment un noyau benzocyclique.

5. Agent selon la revendication 4, caractérisé en ce que le mélange pour la mise en évidence d'estérase et/ou de protéase utilisé est une combinaison d'un sel de diazonium de formule générale I

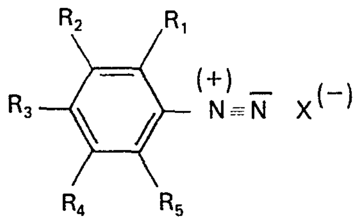

(I),

dans laquelle
$R_1$ est un groupe alkyl inférieur, alcoxy inférieur, alkylmercapto inférieur, N-morpholino, N-thiomorpholino, N-pyrrolidino, N-pipérazino éventuellement N'-alkylé, N-pipéridino, de l'halogène ou de l'hydrogène,
$R_3$ est un groupe alkyle inférieur, alcoxy inférieur, aryloxy, alkylmercapto inférieur, alkylamino, dialkylamino, hydroxyle, N-morpholino, N-thiomorpholino, N-pyrrolidino, N-pipérazino éventuellement N'-alkylé, N-pipéridino, phénylamino, phényle éventuellement substitué par un reste alkyle inférieur ou alcoxy inférieur, de l'halogène ou de l'hydrogène,
$R_2$, $R_4$, $R_5$ pouvant être identiques ou différents sont chacun un groupe alkyle inférieur, alcoxy inférieur, alkylmercapto inférieur, de l'halogène ou de l'hydrogène, $R_1$ et $R_2$ pouvant former ensemble un noyau benzocyclique et
X est un anion stabilisant,
et d'un ester, choisi dans le groupe des composés de formule générale II

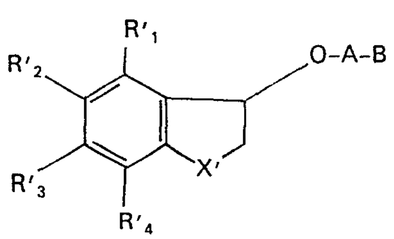

(II),

dans laquelle
$R'_1$, $R'_2$, $R'_3$, $R'_4$ pouvant être selon les cas identiques ou différents, sont chacun un atome d'hydrogène ou d'halogène ou d'halogène, un groupe alkyle inférieur, alcoxy inférieur, aryle, aralkyle, aralcoxy, hydroxyle, carboxyle, carboxy-alcoxy inférieur, aralcoxycarbonyle, aralcoxycarbonyl-alcoxy inférieur, nitro ou acylamino inférieur, ou bien chaque fois deux substituants voisins forment un reste benzocyclique éventuellement substitué par de l'halogène,
X' est un atome de soufre ou un groupe imino éventuellement substitué par un reste alkyle inférieur, aryle, aralkyle ou acyle, A est un reste d'un acide aminé ou d'un peptide
B est un groupe de protection de l'azote habituellement utilisé dans la chimie des peptides ou un dérivé d'un tel groupe
ou des composés de formule générale III

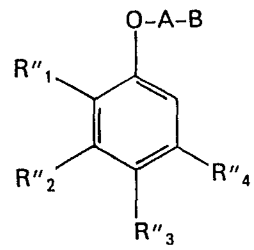

(III),

dans laquelle
$R''_1$, $R''_2$, $R''_3$, $R''_4$, pouvant être identiques ou différents, sont chacun de l'hydrogène, un groupe alkyle inférieur, alcoxy inférieur, alkylamino ou dialkylamino, deux substituants voisins pouvant également former un noyau benzocyclique,
A et B ont la signification indiquée ci-dessus à l'exception de B ne pouvant être un groupe acétyl on benzyle-oxycarbonyle si $R''_1$ et $R''_2$ ou $R''_2$ et $R''_3$ forment un noyau benzocyclique.

6. Agent selon la revendication 4 caractérisé en ce qu'on utilise comme agents auxiliaires complémentaires des agents mouillants, des stabilisants, des activateurs, des agents filmogènes, des additifs galéniques et/ou des formateurs de structures.

7. Les esters d'acides phénoxy-aminés et de peptides de formule générale III

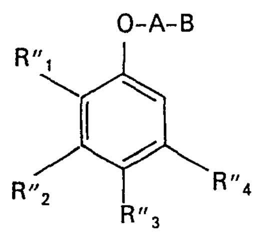

(III),

dans laquelle
$R''_1$, $R''_2$, $R''_3$, $R''_4$, pouvant être identiques ou différents, sont chacun de l'hydrogène, un groupe alkyle inférieur, alcoxy inférieur, alkylamino ou dialkylamino, deux substituants voisins pouvant également former un noxau benzocyclique,
A et B ont la signification indiquée ci-dessus à l'exception de B ne pouvant être un groupe acétyl ou benzyle-oxycarbonyle si $R''_1$ et $R''_2$ ou $R''_2$ et $R''_3$ forment un noyau benzocyclique.

8. Procédé de préparation d'esters d'acides phénoxy-aminés et d'esters de peptides selon la revendication 7 caractérisé en ce qu'on fait réagir des composés de formule générale IV

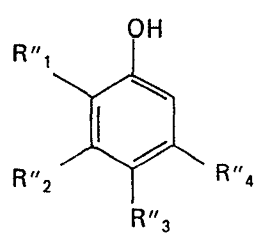

(IV),

dans laquelle
R″$_1$, R″$_2$, R″$_3$ et R″$_4$ ont la signification donnée ci-dessus, avec un acide aminé ou un peptide de formule générale V

HO-A-B (V),

dans laquelle
A et B ont la signification donnée ci-dessus, ou avec des dérivés réactifs appropriés de ces composés selon une des méthodes habituellement pratiquées dans la chimie des peptides.

9. Utilisation de composés selon la revendication 7 pour la préparation d'agents selon la revendication 4.

## Claims

1. Use of agents for the detection of esterolytic and/or proteolytic enzymes consisting of an absorbent carrier, a film layer, a powder mixture, a lyophilisate, a solution or a reagent tablet, containing a suitable esterase and/or protease detection principle, a buffer, as well as possibly further additional materials usually employed for the detection of leucocytes in urine, the agent thereby being added to the urine to be investigated and the resulting colour formation being assessed visually or photometrically, characterised in that as esterase and/or protease detection principle there is used a combination of a specially substituted diazonium salt and a suitable ester, the reactivity of which is adapted to one another by suitable choice of the substituents in such a manner that a sufficiently rapid coupling with the alcohol component liberated from the ester admittedly takes place but no reaction with other components of the test solution, whereby the ester is selected from the group of compounds of the general formula II

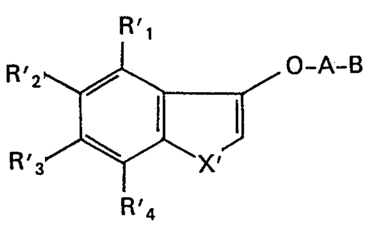

(II)

in which R′$_1$, R′$_2$, R′$_3$, R′$_4$, which can be the same or different, each signify a hydrogen or halogen atom, a lower alkyl, a lower alkoxy, an aryl, an aralkyl, an aralkoxy, a hydroxyl, a carboxy, a carboxy-lower alkoxy, an aralkoxycarbonyl, an aralkoxycarbonyl-lower alkoxy, a nitro or a lower acylamino group or two neighbouring substituents an annellated benzo residue possibly substituted by halogen, X′ a sulphur atom or an imino group possibly substituted by a lower alkyl, an aryl, an aralkyl or an acyl radical, A an amino acid or peptide residue, B a nitrogen protective group usual in peptide chemistry or derived therefrom, or from compounds of the general formula III

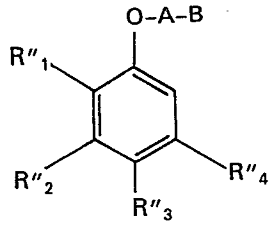

(III),

in which R″$_1$, R″$_2$, R″$_3$, R″$_4$, which can be the same or different, each signify hydrogen, a lower alkyl, a lower alkoxy, alkylamino or dialkylamino group, whereby two neighbouring substituents can also represent an annellated benzene ring, A and B have the above-given meaning.

2. Use of agents according to claim 1, characterised in that the esterase and/or protease detection principle comprises as a diazonium salt a compound of the general formula I

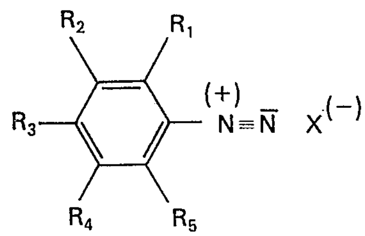

(I),

in which R$_1$ signifies a lower alkyl, a lower alkoxy, a lower alkylmercapto, an N-morpholino, an N-thiomorpholino, an N-pyrrolidino, an optionally N′-alkylated N-piperazino, an N-piperidino group, halogen or hydrogen, R$_3$ a lower alkyl, a lower alkoxy, an aryloxy, a lower alkylmercapto, alkylamino, dialkylamino, a hydroxyl, an N-morpholino, N-thiomorpholino, N-pyrrolidino, a possibly N′-alkylated N-piperazino, N-piperidino, phenylamino, a phenyl group possibly substituted with a lower alkyl or lower alkoxy radical, halogen or hydrogen, R$_2$, R$_4$, R$_5$, which can be the same or different, each a lower alkyl, a lower alkoxy, a lower alkylmercapto group, halogen or hydrogen, whereby R$_1$ and R$_2$ can together represent an annellated benzene ring, and X a stabilising anion.

3. Agent according to claim 1, characterised in that, as additional adjuvant materials, there are used wetting agents, stabilisers, activators, film formers, galenical additive materials and/or structure formers.

4. Agent for the detection of esterolytic and/or proteolytic enzymes in leucocytes in urine consisting of an absorbent carrier, a film layer, a powder mixture, a lyophilisate, a solution or a reagent tablet, containing a suitable esterase and/or protease detection principle, a buffer, as well as possibly further additional materials usually employed, characterised in that as esterase and/or protease detection principle there is used a combination of a specially substituted diazonium salt and a suitable ester, the reactivity of

which is adapted to one another by suitable choice of the substituents in such a manner that a sufficiently rapid coupling with the alcohol component liberated from the ester admittedly takes place but no reaction with other components of the test solution, whereby the ester is selected from the group of compounds of the general formula II

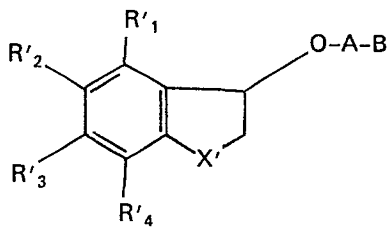

(II),

in which R′$_1$, R′$_2$, R′$_3$, R′$_4$, which can be the same or different, each signify a hydrogen or halogen atom, a lower alkyl, a lower alkoxy, an aryl, an aralkyl, an aralkoxy, a hydroxyl, a carboxy, a carboxy-lower alkoxy, an aralkoxycarbonyl, an aralkoxycarbonyl-lower alkoxy, a nitro or a lower acylamino group or two neighbouring substituents an annellated benzo residue possibly substituted by halogen, X′ a sulphur atom or an imino group possibly substituted by a lower alkyl, an aryl, an aralkyl or an acyl radical, A an amino acid or peptide residue, B a nitrogen protective group usual in peptide chemistry or derived therefrom, or from compounds of the general formula III

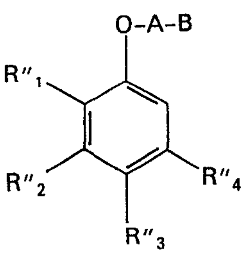

(III),

in which R″$_1$, R″$_2$, R″$_3$, R″$_4$, which can be the same or different, each signify hydrogen, a lower alkyl, a lower alkoxy, alkylamino or dialkylamino group, whereby two neighbouring substituents can also represent an annellated benzene ring, A and B have the above-given meaning, thereby, if R″$_1$ and R″$_2$ and R″$_3$ represent an annellated benzene ring, B not signifying an acetyl- or benzyloxycarbonyl group.

5. Agent according to claim 4, characterised in that, as esterase and/or protease detection principle, there is used a combination of a diazonium salt of the general formula I

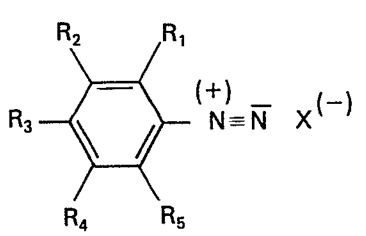

(I),

in which $R_1$ signifies a lower alkyl, a lower alkoxy, a lower alkylmercapto, an N-morpholino, an N-thiomorpholino, an N-pyrrolidino, an optionally N′-alkylated N-piperazino, an N-piperidino group, halogen or hydrogen, $R_3$ a lower alkyl, a lower alkoxy, an aryloxy, a lower alkylmercapto, alkylamino, dialkylamino, a hydroxyl, an N-morpholino, N-thiomorpholino, N-pyrrolidino, a possibly N′-alkylated N-piperazino, N-piperidino, phenylamino, a phenyl group possibly substituted with a lower alkyl or lower alkoxy radical, halogen or hydrogen, $R_2$, $R_4$, $R_5$, which can be the same or different, each a lower alkyl, a lower alkoxy, a lower alkylmercapto group, halogen or hydrogen, whereby $R_1$ and $R_2$ can together represent an annellated benzene ring, and X a stabilising anion, and an ester selected from the group of compounds of the general formula II

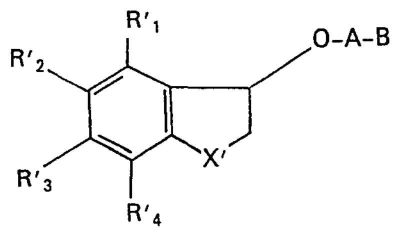

(II),

in which R′$_1$, R′$_2$, R′$_3$, R′$_4$, which can be the same or different, each signify a hydrogen or halogen atom, a lower alkyl, a lower alkoxy, an aryl, an aralkyl, an aralkoxy, a hydroxyl, a carboxy, a carboxy-lower alkoxy, an aralkoxycarbonyl, an aralkoxycarbonyl-lower alkoxy, a nitro or a lower acylamino group or two neighbouring substituents an annellated benzo residue optionally substituted by halogen, X′ a sulphur atom or an imino group possibly substituted by a lower alkyl, an aryl, an aralkyl or an acyl radical, A an amino acid or peptide residue, B a nitrogen protective group usual in peptide chemistry or derived therefrom, or from compounds of the general formula III

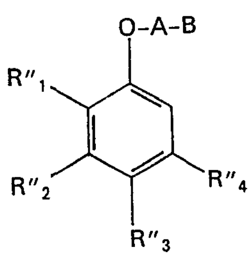

(III),

in which R″$_1$, R″$_2$, R″$_3$, R″$_4$, which can be the same or different, each signify hydrogen, a lower alkyl, a lower alkoxy, alkylamino or dialkylamino group, whereby two neighbouring substituents can also represent an annellated benzene ring, A and B have the above-given meaning, thereby, if R″$_1$ and R″$_2$ or R″$_2$ and R″$_3$ represent an annellated benzene ring, B not signifying an acetyl- or benzyloxycarbonyl group.

6. Agent according to claim 4, characterised in that, as additional adjuvant materials, there are

used wetting agents, stabilisers, activators, film formers, galenical additive materials and/or structure formers.

7. Phenoxyamino acid esters and peptide esters of the general formula III

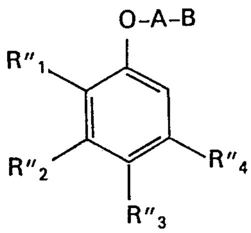

(III),

in which $R''_1$, $R''_2$, $R''_3$, $R''_4$, which can be the same or different, each signify hydrogen, a lower alkyl, a lower alkoxy, alkylamino or dialkylamino group, whereby two neighbouring substituents can also represent an annellated benzene ring, A and B have the above-given meaning, thereby, if $R''_1$ and $R''_2$ or $R''_2$ and $R''_3$ represent an annellated benzene ring, B not signifying an acetyl- or benzyloxycarbonyl group.

8. Process for the preparation of phenoxyamino acid esters and peptide esters according to claim 7, characterised in that one reacts compounds of the general formula IV

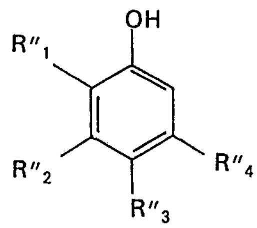

(IV),

in which $R''_1$, $R''_2$, $R''_3$, $R''_4$ have the above-given meaning, with amino acids and peptides of the general formula V

HO-A-B (V),

in which A and B have the above-given meaning, or suitable reactive derivatives thereof according to methods usual in peptide chemistry.

9. Use of compounds according to claim 7 for the preparation of agents according to claim 4.